Europäisches Patentamt

European Patent Office

Office européen des brevets

(11) Veröffentlichungsnummer: **0 325 190**
**A2**

## (12) EUROPÄISCHE PATENTANMELDUNG

(21) Anmeldenummer: 89100670.2

(51) Int. Cl.⁴: **C12N 5/00**

(22) Anmeldetag: 16.01.89

(30) Priorität: 18.01.88 DE 3801236

(43) Veröffentlichungstag der Anmeldung:
26.07.89 Patentblatt 89/30

(84) Benannte Vertragsstaaten:
AT BE CH DE ES FR GB GR IT LI LU NL SE

(71) Anmelder: BOEHRINGER MANNHEIM GmbH
Sandhofer Strasse 112-132
D-6800 Mannheim Waldhof(DE)

(72) Erfinder: Stockinger, Hubertus, Dr. rer. nat.
Unterholzstrasse 22
D-8122 Penzberg(DE)

(74) Vertreter: Huber, Bernhard, Dipl.-Chem. et al
Möhlstrasse 22 Postfach 860 820
D-8000 München 86(DE)

(54) **Pentosansulfat-Medium.**

(57) Ein serumfreies Kulturmedium für tierische und humane Zellen, insbesondere nicht adhärente Zellen, enthält Basismedium und

2 bis 10 mg/l Transferrin,

2 bis 10 mg/l Insulin

1 bis 10 g/l Pepton,

10 bis 500 mg/l $\beta$-D-Xylopyranose, substituiert mit Phosphat-, Carboxy und/oder Sulfatgruppen,

0,1 bis 0,5 mg/l Selenit und

0,1 bis 2 mg/l biologisches Polyamin.

EP 0 325 190 A2

## Pentosansulfat-Medium

Die Kultur von tierischen und humanen Zellen, insbesondere zur Herstellung von Zellprodukten, wie Proteinen über rekombinanter DNA, gewinnt immer größere Bedeutung. Wenn diese Zellkultur im industriellen Maßstab durchgeführt wird, werden große Mengen an Kulturmedien benötigt.

Als Kulturmedien werden bisher die verschiedensten Medien verwendet, z.B. DMEM-Medium (Morton, H.J. (1970) In Vitro 6, 89), F12-Medium (Ham, R.G. (1965) Proc.Natl. Acad.Sci. USA 53, 288) und RPMI 1640-Medium (Goding, J.W. (1980) J.Immunol.Methods 39, 285, JAMA 199 (1957) 519). All diesen Medien muß jedoch dann, wenn Zellen (nichtadhärente, anchorage independent Zellen) in Suspension gezüchtet werden sollen, Serum zugefügt werden. In der Regel wird hierzu fötales Kälberserum, Pferdeserum oder humanes Serum in einer Konzentration von 1 bis 15 %, üblicherweise 5 % verwendet. Diese Seren sind jedoch teuer und in großen Mengen kaum beschaffbar. Eine Verringerung der Konzentration im Medium konnte bisher nicht erreicht werden, da die Zellen dann in immer stärkerem Maße verklumpen. Die Zellaggregation ist dann am stärksten ausgeprägt, wenn die Zellen serumfrei kultiviert werden. Derartig verklumpte Zellen sterben rasch ab, wodurch die Ausbeute an dem gewünschten Zellprodukt drastisch abnimmt.

Aus diesem Grund besteht ein großes Interesse an serumfreien Medien, in denen nichtadhärente Zellen ohne Verklumpung gezüchtet werden können. So ist beispielsweise in In vitro Cell.Dev.Biol. 21 (1985) 588-592, ein serumfreies Medium beschrieben, welches zur Kultivierung von CHO-Zellen geeignet sein Soll und eine 1:1 Mischung von Ham's F12-Medium und DMEM enthält, welchem Transfer rin, Insulin und Selenit zugegeben wurde. Weitere bekannte Medien enthalten außer dem Basismedium Zusätze von biologischen Polyaminen (J.Biol.Chem. 261 (1986) 9502-9508) bzw. Bacteropeptone und Insulin (Biotechnol. Bioeng. 18 (1976) 363-382) bzw. Ornithin und/oder Polyamin (Mol. Cell.Biol. 4 (1984) 915-922).

Diese bekannten serumfreien Medien haben jedoch Nachteile. So ist es häufig erforderlich, während der Kultur der Zellen in gewissen Zeitabständen mit fötalem Kälberserum nachzufüttern. Ebenso ist die Übertragung der Stammkultur, die üblicherweise in serumhaltigem Medium gehalten wird, in das serumfreie Medium nur durch langsame Adaptation möglich.

Der vorliegenden Erfindung liegt daher die Aufgabe zugrunde, ein serumfreies Medium bereitzustellen, welches für das Wachstum von nicht adhärenten Zellen geeignet, unproblematisch zu handhaben und kostengünstig ist.

Diese Aufgabe wird erfindungsgemäß gelöst durch ein serumfreies Kulturmedium für tierische und humane Zellen, insbesondere nichtadhärente Zellen, enthaltend Basismedium, Transferrin, Insulin und Selenit, welches dadurch gekennzeichnet ist, daß es

2 bis 10 mg/l Transferrin,

2 bis 10 mg/l Insulin

1 bis 10 g/l Pepton,

10 bis 500 mg/l β-D-Xylopyranose, substituiert mit Phosphat-, Carboxy- und/oder Sulfatgruppen,

0,1 bis 0,5 mg/l Selenit und

0,1 bis 2 mg/l biologisches Polyamin enthält oder daraus besteht.

Die Erfindung beruht auf der überraschenden Entdeckung, daß ein serumfreies Kulturmedium für tierische und humane Zellen, das die Nachteile der bisher bekannten Medien nicht aufweist, erhalten ist, wenn es einen bestimmten Gehalt an β-D-Xylopyranose, substituiert mit Phosphat-, Carboxy- und/oder Sulfatgruppen aufweist. Derartige β-D-Xylopyranosen sind bekannt und handelsüblich und beispielsweise unter den Bezeichnungen Pentosanpolysulfat, Xylanhydrogensulfat, Xylanpolysulfat, CB 8061, Fibrase, Hemoklar, SP 54 (Natriumsalz) und Thrombozit (Natriumsalz) erhältlich.

Xylopyranosen sind von Xylose abstammende ringförmige Zucker.

Bevorzugt werden β-D-Xylopyranosen mit einem Molekulargewicht von 1.000 bis 10.000 Dalton, besonders bevorzugt mit einem Molekulargewicht von 4.000 bis 6.000 Dalton verwendet. Ganz besonders bevorzugt wird Pentosanpolysulfat eingesetzt. Die Konzentration der ß-D-Xylopyranose beträgt vorzugsweise 50 bis 200 mg/l, besonders bevorzugt 80 bis 120 mg/l.

Als Basismedien sind alle bekannten Medien zur Zellkultur geeignet, z.B. DMEM-Medium, F12-Medium und RPMI 1640-Medium und wie sie z.B. von Boehringer Mannheim GmbH oder Gibco BRL Life Technology, USA unter diesen Bezeichnungen vertrieben werden. Bevorzugt wird aber eine Mediummischung von DMEM/F12 im Verhältnis 1 : 1.

Transferrin wird dem Medium in einer Konzentration von 2 bis 10 mg/l vorzugsweise 4 bis 6 mg/l zugesetzt. Die Konzentration in Insulin beträgt 2 bis 10 mg/l vorzugsweise 4 bis 6 mg/l. Als Pepton wird vorzugsweise pflanzliches Pepton, besonders bevorzugt aus Sojabohnen, in einer Konzentration von 1 bis 10 g/l, vorzugsweise 1 bis 4 g/l verwendet.

Geeignete biologische Polyamine sind beispielsweise Putrescin, Spermidin, Spermin oder Ornithin. Bevorzugt wird Putrescin verwendet. Die Konzentration des Polyamins beträgt 0,1 bis 2 mg/l, vorzugsweise 0,5 bis 1,5 mg/l. Selenit wird in einer Konzentration von 0,1 bis 0,5 mg/l, vorzugsweise 0,1 bis 0,3 mg/l zugesetzt. Wenn transfektierte Zellen gezüchtet werden sollen, welche Vektoren mit Resistenzgenen enthalten, wird zur Aufrechterhaltung der Stabilität der Plasmidtransfektion dem Medium zusätzlich ein dem im Vektor enthaltenen Resistenzgen entsprechendes Selektionsmittel hinzugefügt. Geeignete Selektionsmittel sind dem Fachmann bekannt, beispielsweise Neomycin, Hygromycin, Mycophenolsäure, Hypoxanthin, Xanthin, Aminopterin oder aber auch Methotrexat und Derivate.

Mit dem erfindungsgemäßen Medium können humane und tierische Zellen, sowohl adhärente als auch Suspensionszellen, serumfrei kultiviert werden. Besonders geeignet hierfür sind:
Bowes Melanoma Zellen (Biochem.J. 336 (1985) 631-636), menschliche Endothelzellen (J.Lab.Clin.Med. 109 (1987) 97-104), Sertoli Zellen (Ratte) (Biol.Reprod. 34 (1986) 895-904), Keratozyten (Meerschwein) (Dev.Biol.Stand. 60 (1985) 439-446), Epithelzellen (Mensch, Kuh, Kaninchen) (Exp.Eye Res. 42 (1986) 417-431), Carcinoma Zellen (Meerschwein) (Cancer Res. 43 (1983) 1783-1789), CHO Zellen (J.Mol. Biol. 159 (1982) 601-621) und Maus- bzw. menschliche Hybridoma-Zellen.

Die nachfolgenden Beispiele erläutern die Erfindung weiter.

Beispiel 1:

a) Zellkultivierung

CHO-Zellen (ATCC CCL 61, CHO K1) wurden in serumfreier Suspensionskultur gezüchtet. Das Kulturmedium (Mischung von DMEM und Hams's F12-Medium 1:1) enthält zusätzlich 5 mg/l Transferrin, 5 mg/l Insulin, 1 mg/l Putrescin, 0,2 mg/l Selenit und 2 g/l Pepton aus Sojabohnen und 100 mg/l Pentosanpolysulfat.

Es werden $10^5$ Zellen pro ml Medium ausgesät, in Roller-oder Spinnerflaschen gefüllt und bis zum Erreichen der maximalen Zelldichte kultiviert ($10^6$ Zellen/ml nach 120 Stunden).

b) Bestimmung der Lebendkeimzahl und der Lactatdehydrogenase-Aktivität in Zellkulturen mit und ohne Pentosanpolysulfat

Es wird analog Beispiel 1 kultiviert und die Zellzahl und LDH-Aktivität als Maß für die Menge abgestorbener Zellen, die LDH verlieren, zu verschiedenen Zeiten bestimmt. Die Ergebnisse sind aus Tabelle 1 zu ersehen:

Tabelle 1

| Bestimmung der Lebendzellzahl und Lactatdehydrogenase (LDH) in Zellkulturen mit und ohne Pentosanpolysulfat | | | | |
|---|---|---|---|---|
| Tage | ohne Pentosanpolysulfat | | mit Pentosanpolysulfat | |
| | Zellzahl x $10^5$ | LDH(u/l) | Zellzahl x $10^5$ | LDH(u/l) |
| 0 | 2,5 | 0 | 1 | 0 |
| 1 | 5,7 | 82 | 1,5 | 13 |
| 2 | 4,5 | 120 | 2,2 | 13 |
| 3 | 2,5 | 146 | 4,3 | 15 |
| 4 | 2,8 | 150 | 6,5 | 28 |
| 5 | 3,2 | 228 | 8,7 | 36 |

Es zeigt sich, daß mit Pentosanpolysulfat sowohl eine höhere Zellzahl, als auch ein geringeres Absterben der Zellen (geringere LDH-Aktivität) beobachtet wird. Analoge Ergebnisse werden mit beliebigen

3

EP 0 325 190 A2

CHO-Zellinien erhalten.

<u>Beispiel 2</u>

Maus-Hybridoma-Zellen (ECACC 84122002, DE 3507849.9) werden in serumfreiem RPMI 1640-Medium kultiviert. Das Medium enthält zusätzlich 5 mg/l Transferrin, 5 mg/l Insulin, 1 mg/l Putrescein, 0,2 mg/l Selenit, 2 g/l Pepton aus Sojabohnen und 100 mg/l Pentosanpolysulfat.

Wie in Beispiel 1 beschrieben, werden die Zellen ausgesät und die Menge der gebildeten FSH-Antikörper über einem ELISA-Test bestimmt.

Tabelle 2 zeigt das Zellwachstum und die Antikörperproduktion.

| Tage | Zellzahl (x $10^5$) | Antikörper (mg/l) |
|---|---|---|
| 0 | 1 | 0 |
| 1 | 0,95 | 9,8 |
| 2 | 2,4 | 14,0 |
| 3 | 5,2 | 26,0 |
| 4 | 9,8 | 41,0 |
| 7 | 5,9 | 46,0 |

<u>Beispiel 3</u>

LMTK$^-$-Zellen (ATCC CCL 1.3) werden in serumfreier Kultur gezüchtet. Das Kulturmedium ist mit dem in Beispiel 1 beschriebenen Medium identisch. Es werden $10^5$ Zellen pro ml Medium ausgesät und bis zum Erreichen der maximalen Zelldichte kultiviert ($10^6$ Zellen/ml nach 120 Stunden).

Es zeigt sich, daß mit Pentosanpolysulfat sowohl eine höhere Zellzahl als auch ein geringeres Absterben der Zellen (geringere LDH-Aktivität) beobachtet wird.

## Ansprüche

1. Serumfreies Kulturmedium für tierische und humane Zellen, insbesondere nicht adhärente Zellen, enthaltend Basismedium, Transferrin, Insulin und Selenit, welches
**dadurch gekennzeichnet** ist, daß es
2 bis 10 mg/l Transferrin,
2 bis 10 mg/l Insulin
1 bis 10 g/l Pepton,
10 bis 500 mg/l $\beta$-D-Xylopyranose, substituiert mit Phosphat-, Carboxy und/oder Sulfatgruppen,
0,1 bis 0,5 mg/l Selenit und
0,1 bis 2 mg/l biologisches Polyamin enthält oder daraus besteht.

2. Kulturmedium nach Anspruch 1,
**dadurch gekennzeichnet,** daß die $\beta$-D-Xylopyranose ein Molekulargewicht von 1.000 bis 10.000 Dalton aufweist.

3. Kulturmedium nach Anspruch 2,
**dadurch gekennzeichnet,** daß die $\beta$-D-Xylopyranose ein Molekulargewicht von 4.000 bis 6.000 Dalton aufweist.

4. Kulturmedium nach einem der vorhergehenden Ansprüche,
**dadurch gekennzeichnet,** daß die Konzentration der $\beta$-D-Xylopyranose 50 bis 200 mg/l beträgt.

5. Kulturmedium nach einem der vorhergehenden Ansprüche,
**dadurch gekennzeichnet,** daß die $\beta$-D-Xylopyranose Pentosanpolysulfat ist.

6. Kulturmedium nach einem der vorhergehenden Ansprüche,
**dadurch gekennzeichnet,** daß es als Basismedium DMEM oder/und F12-Medium enthält.

4

7. Kulturmedium nach einem der vorhergehenden Ansprüche,
**dadurch gekennzeichnet,** daß es als biologisches Polyamin mindestens eine Substanz aus der Gruppe Putrescin, Spermidin, Spermin oder Ornithin enthält.

8. Kulturmedium nach einem der vorhergehenden Ansprüche,
**dadurch gekennzeichnet,** daß es 0,1 bis 0,3 mg/l Selenit enthält.

9. Kulturmedium nach einem der vorhergehenden Ansprüche,
**dadurch gekennzeichnet,** daß es ein Selektionsmittel enthält, welches einem Resistenzgen in der zu züchtenden Zelle entspricht.

10. Verwendung eines Kulturmediums nach einem der Ansprüche 1 bis 9 zur Züchtung von Zellen aus der Gruppe Bowes Melanoma-Zellen, menschliche Endothelzellen, Sertoli-Zellen, Keratozyten, Epithelzellen, Carcinoma-Zellen, Hybridoma-Zellen und CHO-Zellen.